# EUROPEAN PATENT SPECIFICATION

(11) **EP 4 285 950 B1**
(45) Date of publication and mention of the grant of the patent: **19.11.2025**
(21) Application number: 22766674.0
(22) Date of filing: 31.01.2022
(51) Int. Cl.: A61L 29/06, A61L 29/12, A61L 29/14, A61M 25/00

(54) **CATHETER AND BALLOON CATHETER**
KATHETER UND BALLONKATHETER
CATHÉTER ET CATHÉTER À BALLONNET

(30) Priority: 12.03.2021 JP 2021039903
(43) Date of publication of application: 06.12.2023
(73) Proprietor: TERUMO Kabushiki Kaisha, Tokyo 151-0072 (JP)
(72) Inventor: FUKUDA, Keiji, Fujinomiya-shi, Shizuoka 418-0015 (JP)
(74) Representative: Casalonga
(86) International application number: PCT/JP2022/003548
(87) International publication number: WO 2022/190707

(56) References cited:
- WO-A1-2011/070844
- JP-A- 2005 046 184
- JP-A- 2009 544 416
- JP-A- 2016 174 827
- JP-A- H0 833 705
- US-A1- 2004 101 644

## Description

### Technical Field

The present invention relates to a catheter and a balloon catheter including a catheter tube having a predetermined layer structure.

### Background Art

In the related art, balloon catheters are used to eliminate and treat constriction of a stenosis in a living body. In the treatment, a balloon is introduced into a stenosis developed in a lumen or coelom, and a balloon catheter is used to expand the stenosis outward from the inside. For example, a balloon catheter is employed in percutaneous transluminal coronary angioplasty (PTCA) in which a balloon is used to expand a stenosis of a coronary artery to improve blood flow. In a balloon catheter, a balloon is connected to a distal portion of an elongated shaft member stretching in an axial direction.

A shaft member of a catheter, or a typical a balloon catheter, includes an outer tube and an inner tube disposed in a lumen of the outer tube. Since the shaft member is introduced by a user (operator) into a stenosis in a living body, the shaft member is required to have sufficient flexibility to ensure trackability within the living body. Furthermore, since a guidewire is inserted through a lumen of the inner tube, the inner tube is required to have a luminal surface provided with slidability that enables the operation of the guidewire.

In manufacturing a catheter, a catheter is typically assembled by adhesion or fusion bonding of resin. For this reason, using the same resin material for an inner tube and members such as a catheter distal portion (distal tip) and a balloon achieves desirable compatibility in the adhesion or fusion bonding and enhances workability of the adhesion or fusion bonding.

Therefore, the following combination has been studied to be used in a catheter: a combination of a polyolefin as a material with excellent slidability and a polyamide as a material with excellent flexibility and preferable to be used in a balloon member or the like.

JP 2016-174827 A (corresponding to US 2016/0271361 A) discloses a technique related to a catheter tube including an inner layer composed of a modified polyolefin resin, a first polyamide layer formed on an outer surface of the inner layer, and a second polyamide layer formed on an outer surface of the first polyamide layer and containing a colorant. US 2004/101644 A1 discloses a balloon catheter made of a composite material wherein the matrix resin is reinforced by short carbon nanotube or metal fibers.

### Summary of Invention

A catheter including the catheter tube disclosed in JP 2016-174827 A (corresponding to US 2016/0271361 A) is excellent in operability since it has a luminal surface provided with excellent slidability and also has desirable flexibility and a desirable burst strength.

On the other hand, recent medical devices have been significantly reduced in size and diameter, and a surgery to approach a medical device with higher pliability toward a narrower lesion site in a living body is becoming popular in medical treatment. For this reason, there is demand for a catheter capable of exhibiting desirable operability even in a complicated lesion site. Specifically, there is demand for a catheter that has not only excellent slidability for enabling the operation of a guidewire but also capabilities to maintain a lumen through which the guidewire is inserted and to prevent kinking.

Accordingly, there is demand for a technique to provide a catheter tube with a desirable burst strength while maintaining slidability of an inner surface.

The invention has been made in light of the problems, and an object of the invention is to provide a catheter that includes a catheter tube having an inner surface with desirable slidability and having an excellent burst strength. Another object of the invention is to provide a balloon catheter including the catheter.

The inventors have intensively studied to solve the problems. As a result of the intensive studies, the inventors have found that the problems are solved by the following catheter: a catheter including a catheter tube that includes a first polyamide layer and a second polyamide layer disposed on an outer surface of the first polyamide layer, the first polyamide layer containing a pressure resistance enhancer in an amount more than 10 wt.% but equal to or less than 30 wt.% of total solids content of the first polyamide layer, wherein the pressure resistance enhancer contains amorphous carbon and a metal compound, wherein the amorphous carbon and metal compound are carbon black and titanium oxide.

### Brief Description of Drawings

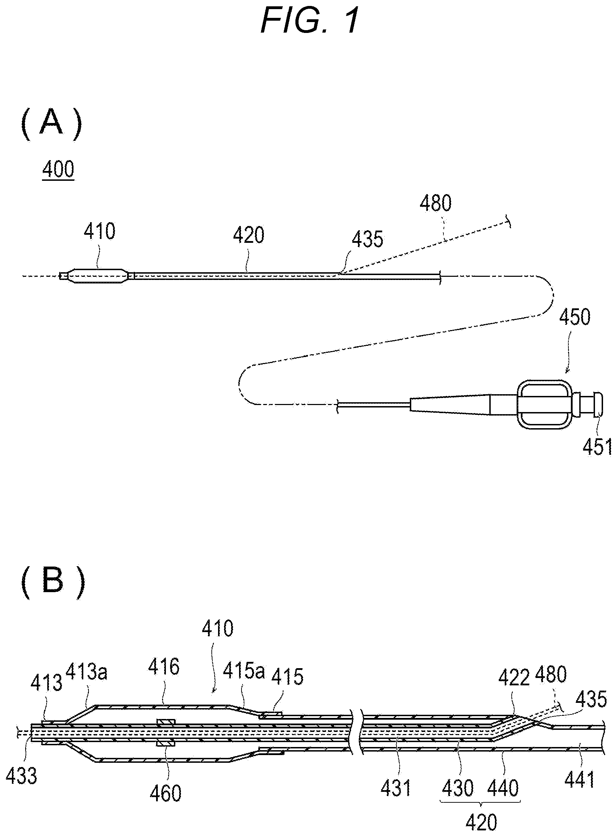

Fig. 1 is a view illustrating a balloon catheter according to an embodiment in which (A) illustrates a simplified overall configuration of the balloon catheter and (B) illustrates an enlarged cross-sectional view of a distal portion of the balloon catheter. In Figs. 1(A) and (B), the reference numeral 410 represents a balloon; the reference numeral 420 represents a shaft; the reference numeral 430 represents an inner tube; the reference numeral 431 represents a guidewire lumen; the reference numeral 440 represents an outer tube; and the reference numeral 450 represents a hub.

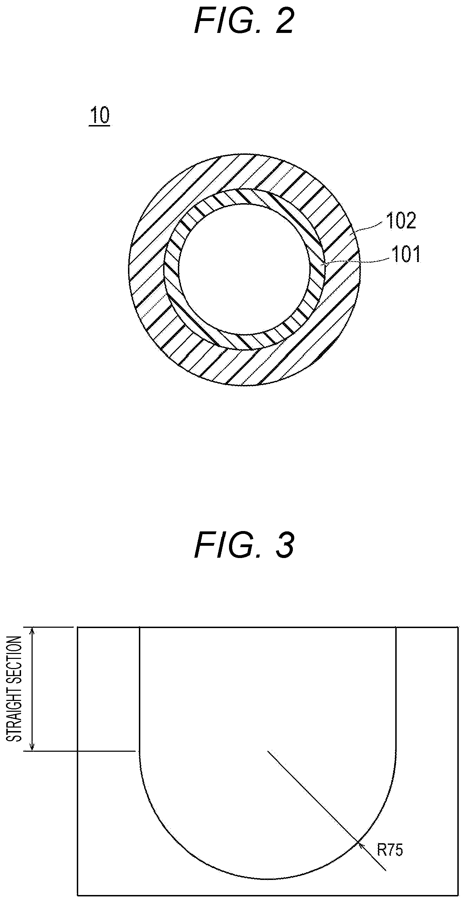

Fig. 2 is a view illustrating a specific example of a layer configuration (cross-sectional view perpendicular to an axis) of a catheter tube according to the invention. In Fig. 2, the reference numeral 10 represents the catheter tube; the reference numeral 101 represents a first polyamide layer; and the reference numeral 102 represents a second polyamide layer.
Fig. 3 is a schematic view for describing a jig used for evaluating slidability of tubes obtained in Examples and Comparative Examples.

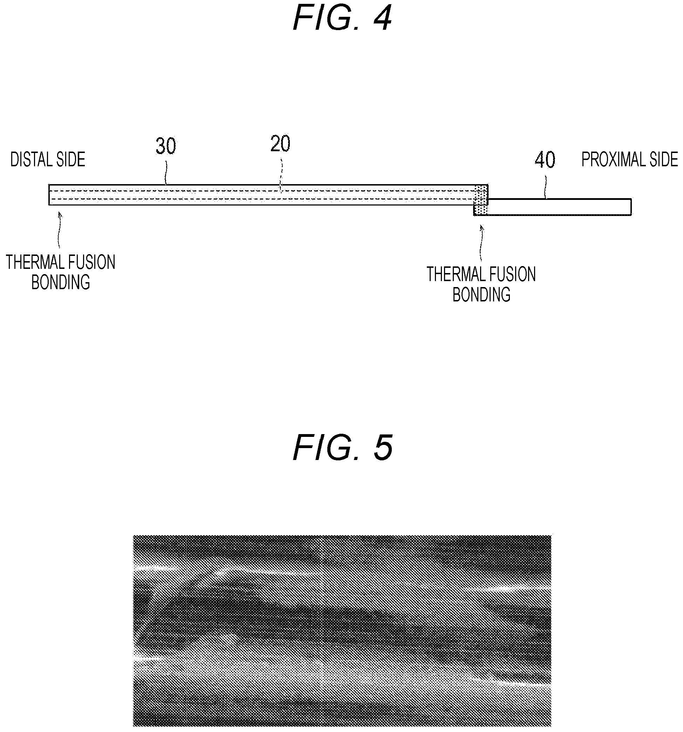

Fig. 4 is a schematic view for describing an evaluation sample used for evaluating the burst strength of each tube obtained in Examples and Comparative Examples. In Fig. 4, the reference numeral 20 represents an inner tube; the reference numeral 30 represents an outer tube; and the reference numeral 40 represents an intermediate shaft.
Fig. 5 is a photograph of a tube obtained in Example 1-1 observed by a CCD camera.

### Description of Embodiments

An embodiment of the invention is a catheter including a catheter tube that includes a first polyamide layer and a second polyamide layer disposed on an outer surface of the first polyamide layer. The first polyamide layer contains a pressure resistance enhancer in an amount more than 10 wt.% but equal to or less than 30 wt.% of total solids content of the first polyamide wherein the pressure resistance enhancer contains amorphous carbon and a metal compound, wherein the amorphous carbon and metal compound are carbon black and titanium oxide. Hereinafter, "the catheter tube that includes the first polyamide layer and the second polyamide layer disposed on the outer surface of the first polyamide layer, in which the first polyamide layer contains the pressure resistance enhancer in an amount more than 10 wt.% but equal to or less than 30 wt.% of total solids content of the first polyamide layer, wherein the pressure resistance enhancer contains amorphous carbon and a metal compound, wherein the amorphous carbon and metal compound are carbon black and titanium oxide" is simply referred to as "the catheter tube according to the invention."

The catheter tube according to the invention includes the first polyamide layer as an inner layer and the second polyamide layer as an outer layer, and the first polyamide layer as the inner layer contains a predetermined amount of the pressure resistance enhancer. The inventors have surprisingly found that a catheter tube with such a configuration has an excellent burst strength while maintaining desirable slidability. Although detailed mechanism is unknown, since the catheter tube according to the invention has the inner layer and the outer layer both formed from polyamide-based resins, the catheter tube not only has good conformity between the resins (adhesiveness between the inner layer and the outer layer) but also tends to have enhanced rigidity due to annealing and fusion bonding, and the inventors have inferred that these effects enhances the burst strength. In addition, the inventors have inferred that these effects are attributed to a certain factor such as the structure (for example, particle structure), component, and amount of the pressure resistance enhancer. Note that the above mechanism is an inference and does not limit the technical scope of the invention at all.

Hereinafter, embodiments of the invention will be described. Note that the invention is not limited to the following embodiments.

Herein, a range from "X to Y" indicates "X or more and Y or less." Unless otherwise specified, operations and measurements of physical properties and the like are performed at room temperature (20 to 25°C) and at relative humidity of 40 to 50% RH. Furthermore, the expression "A and/or B" represents either A or B and also all combinations of A and B. Specifically, the expression represents at least one of A and B, that is, A, B, and combinations of A and B.

### <Catheter>

An embodiment of the invention is a catheter including a catheter tube that includes a first polyamide layer and a second polyamide layer disposed on an outer surface of the first polyamide layer. The first polyamide layer contains a pressure resistance enhancer in an amount more than 10 wt.% but equal to or less than 30 wt.% of total solids content of the first polyamide layer. According to the invention, there is provided a catheter that includes a catheter tube having an inner surface with desirable slidability and having an excellent burst strength.

The catheter tube according to the invention is employed as an inner tube and/or outer tube of a balloon catheter, but it is particularly preferable to use the catheter tube as an inner tube. Hereinafter described are details of a balloon catheter 400, a specific example of the invention illustrated in Fig. 1, but note that the invention is not limited by the following description.

The balloon catheter 400 is used as, for example, a PTCA catheter, a medical device used for treating a stenosis (lesion). In the treatment, a shaft 420 is inserted into a biological organ and a balloon 410 disposed in the distal side of the shaft 420 is inflated at the stenosis to expand the stenosis. The balloon catheter 400 may also be used for other treatments. For example, the balloon catheter 400 may be used to treat and improve a stenosis developed in a biological organ such as a blood vessel other than a coronary artery, the bile duct, the trachea, the esophagus, the urethra, and other organs.

As illustrated in Fig. 1(A), the balloon catheter 400 includes the elongated shaft 420 having flexibility, the balloon 410 disposed in a distal portion of the shaft 420 and capable of inflating and deflating in a deformable manner, and a hub 450 disposed in a proximal portion of the shaft 420. In describing the balloon catheter 400, a side provided with the balloon 410 is referred to as "distal side" and a side provided with the hub 450 is referred to as "proximal side."

The balloon catheter 400 is of what is called a rapid exchange type provided with an opening (proximal opening) 435 disposed closer to the distal portion of the shaft 420. A guidewire 480 is introduced into this opening 435. Alternatively, the balloon catheter 400 may be of what is called an over-the-wire type having a guidewire lumen 431 (see Fig. 1(B)) extending from a distal end to a proximal end of the shaft 420.

As illustrated in Fig. 1(B), the shaft 420 includes an inner tube (inner tube shaft) 430 provided with the guidewire lumen 431 through which the guidewire 480 is inserted and an outer tube (outer tube shaft) 440 provided with a pressurizing medium lumen 441 between the outer periphery of the inner tube 430. To the pressurizing medium lumen 441, a pressurizing medium for inflating the balloon 410 is supplied.

The shaft 420 has a double tube structure in which the inner tube 430 is inserted into the outer tube 440 in a concentric manner.

As illustrated in Fig. 1(B), the inner tube 430 includes two openings, that is, a distal opening 433 formed in a distal end and the proximal opening 435 formed in a proximal end. The guidewire lumen 431 extends inside the inner tube 430 and is communicated with the openings 433 and 435.

The inner tube 430 includes a hollow tube material (catheter tube) having the proximal side curved radially outward. A distal portion 413 of the balloon 410 is joined to the inner tube 430 at a part close to the distal end of the inner tube 430 in a liquid-tight and air-tight manner by a known method such as fusion bonding. In addition, a connection opening 422 formed at a predetermined position of the outer tube 440 is joined to the inner tube 430 at a part close to the proximal end of the inner tube 430 in a liquid-tight and air-tight manner. The guidewire 480 is inserted through the guidewire lumen 431, using the distal opening 433 disposed in the distal end of the inner tube 430 and the proximal opening 435 disposed in the proximal end of the inner tube 430 as an outlet and an inlet, respectively.

A distal tip may be attached to the distal end of the inner tube 430 to protect a biological organ (for example, an inner wall of a blood vessel) from damage when, for example, the distal end of the balloon catheter 400 comes into contact with the biological organ. The distal tip includes, for example, a tubular member more flexible than the inner tube 430. Note that the distal tip preferably includes a polyamide resin, a polyamide elastomer, or a blend thereof. With this configuration, when the distal tip and the inner tube are fusion bonded, it is possible to enhance an adhesion force of the inner tube 430 according to the invention with respect to the distal tip.

In an embodiment of the invention, the catheter tube according to the invention is employed as the inner tube 430. In addition to the following configuration, the catheter tube may contain for example, antithrombotic substances such as heparin, prostaglandins, urokinase, and arginine derivatives.

The outer tube 440 includes a hollow tube material that stretches from near a proximal portion 415 of the balloon 410 to the hub 450 (see Fig. 1(A)). The proximal portion 415 of the balloon 410 is joined to the distal end of the outer tube 440 in a liquid-tight and air-tight manner by a known method such as welding.

Examples of the material used in the outer tube 440 include polyolefins such as polyethylene, polypropylene, ethylene-propylene copolymer, and ethylene-vinyl acetate copolymer, thermoplastic resins such as soft polyvinyl chloride, various rubbers such as silicone rubber and latex rubber, various elastomers such as polyurethane elastomers, polyamide elastomers, and polyester elastomers, and crystalline plastics such as polyamide resin, crystalline polyethylene, and crystalline polypropylene. The outer tube 440 may be formed from a polyamide-based resin to be described. In addition, a part of the outer tube 440 that comes into contact with blood (for example, an outer surface of the outer tube 440) may be coated with an antithrombotic substance or formed from an antithrombotic substance-containing resin.

The balloon 410 is preferably formed from an elastic material. Examples of the material include thermoplastic elastomers such as vinyl chloride, polyurethane elastomers, polystyrene elastomers, styrene-ethylene-butylene-styrene copolymer (SEBS), and styrene-ethylene-propylene-styrene copolymer (SEPS), thermoplastic resins such as PET, thermosetting resins such as rubber and silicone elastomers, and polyamide-based resins such as polyamide resin, polyamide elastomers, or a blend thereof. Alternatively, the balloon 410 may be a multilayer balloon having two or more layers. Note that the balloon 410 is preferably formed from a polyamide resin, a polyamide elastomer, or a blend thereof. Accordingly, in a case where the catheter tube according to the invention is used as, for example, an inner tube, it is possible to enhance an adhesive force between a balloon and an inner tube when the balloon and the inner tube are fusion bonded.

As illustrated in Fig. 1(A), the hub 450 includes a connection 451 connectable in a liquid-tight and air-tight manner to a supplying device such as an indeflator (not illustrated) for supplying a pressurizing medium. The connection 451 of the hub 450 may be, for example, a known Luer taper which a fluid tube or the like can be connected to or separated from.

The pressurizing medium (for example, saline and contrast) used for inflation of the balloon 410 flows into the shaft 420 through the connection 451 of the hub 450. The pressurizing medium is supplied to the balloon 410 via the pressurizing medium lumen 441.

As illustrated in Fig. 1(B), the balloon 410 includes an effective inflation portion (pressurizing portion) 416 that inflates in a deformable manner to expand a stenosis, a distal tapered portion 413a continuous with the distal side of the effective inflation portion 416, and a proximal tapered portion 415a continuous with the proximal side of the effective inflation portion 416. The distal portion 413 placed in the distal side of the distal tapered portion 413a is fixed to an outer surface of the inner tube 430, and the proximal portion 415 placed in the proximal side of the proximal tapered portion 415a is fixed to the outer surface of the outer tube 440. A part of the inner tube 430 corresponding to a central portion of the effective inflation portion 416 is provided with an X-ray contrast marker 460 for indicating the central portion of the effective inflation portion 416. Alternatively, the X-ray contrast marker 460 may be disposed in parts of the inner tube 430 that correspond to both ends of the effective inflation portion 416 for indicating the both ends of the effective inflation portion 416.

In a preferred embodiment of the invention, the catheter tube according to the invention is employed as an inner tube of a balloon catheter. Furthermore, in a preferred embodiment, the balloon catheter according to the invention includes a balloon containing a polyamide-based resin, and the catheter tube according to the invention is employed as an inner tube of the balloon catheter. In other words, in an embodiment of the invention, there is provided a balloon catheter including an outer tube having a lumen, an inner tube disposed in the lumen of the outer tube, and a balloon fixed to the distal side of the inner tube and the distal side of the outer tube, in which the balloon contains a polyamide-based resin, the inner tube includes a first polyamide layer and a second polyamide layer disposed on an outer surface of the first polyamide layer, and the first polyamide layer contains a pressure resistance enhancer in an amount more than 10 wt.% but equal to or less than 30 wt.% of total solids content of the first polyamide layer. As the polyamide-based resin contained in the balloon, examples to be recited in the description of the first polyamide layer may be employed. The polyamide-based resin used as a forming material of the balloon may be the same or different from the polyamide-based resin used as a forming material of the first polyamide layer or the second polyamide layer. In an embodiment of the invention, the balloon is substantially composed of a polyamide-based resin.

Fig. 2 illustrates a specific example of a layer configuration (cross-sectional view perpendicular to an axis) of the catheter tube according to the invention, but the specific example illustrated in Fig. 2 does not limit the invention. In Fig. 2, the catheter tube has a circular cross-sectional shape perpendicular to the axis but the cross-sectional shape of the catheter tube is not limited thereto and may be polygonal. In Fig. 2, a catheter tube 10 includes a first polyamide layer (inner layer) 101 and a second polyamide layer (outer layer) 102 formed on an outer surface of the first polyamide layer 101. In a specific example illustrated in Fig. 2, the catheter tube 10 includes the second polyamide layer (outer layer) 102 larger in thickness than the first polyamide layer (inner layer) 101. Making the colorant-free second polyamide layer (outer layer) 102 thicker than the pressure resistance enhancer-containing first polyamide layer (inner layer) 101 enhances the tensile strength of the catheter tube 10. Accordingly, when the catheter tube 10 is employed as an inner tube or outer tube of a balloon catheter, it is possible to enhance the strength of an adhered part between the catheter tube 10 and a resin member such as a distal tip and a balloon that contains a polyamide resin, a polyamide elastomer, or a blend thereof. In other words, the apparent adhesiveness is enhanced.

In addition to the first polyamide layer (inner layer) 101 and the second polyamide layer (outer layer) 102, the catheter tube 10 according to the invention may optionally include the following layers.

For example, in order to further enhance the slidability of the guidewire, the catheter tube according to the invention may also include a fluorine-based resin layer composed of a fluorine-based resin such as polytetrafluoroethylene, polyvinylidene fluoride, ethylene tetrafluoroethylene, or perfluoroalkoxy resin (not illustrated) in an inner surface of the first polyamide layer (inner layer) 101. However, from a perspective of size reduction and diameter reduction of a catheter tube, the catheter tube 10 according to the invention preferably includes the first polyamide layer (inner layer) 101 and the second polyamide layer (outer layer) 102.

The outside diameter of the catheter tube according to the invention is set appropriately depending on the intended use and the catheter tube 10 is not particularly limited in outside diameter. When employed as an inner tube, the catheter tube 10 has an outside diameter of, for example, 300 to 800 µm, and preferably, 500 to 600 µm. Furthermore, the catheter tube is not particularly limited in inner diameter. When employed as an inner tube, the catheter tube 10 has an inner diameter of, for example, 250 to 600 µm, and preferably, 400 to 500 µm.

The catheter tube according to the invention has a thickness of (a thickness of all the layers including the first polyamide layer, second polyamide layer, and other optional layers), for example, 50 to 100 µm, and preferably, 60 to 80 µm.

Hereinafter described are details of the first polyamide layer (inner layer) and the second polyamide layer (outer layer).

### [First Polyamide Layer]

In an embodiment of the invention, the first polyamide layer (inner layer) contains a polyamide-based resin and a pressure resistance enhancer in a predetermined amount. In a preferred embodiment of the invention, the first polyamide layer (inner layer) is composed of a polyamide-based resin and a pressure resistance enhancer in a predetermined amount. Polyamide-based resins are flexible, having a high strength, and offering an advantage of a larger elastic region than polyesters such as polyethylene terephthalate. In addition, polyamide-based resins have a relatively high melting point and can be heated at high temperatures during annealing or fusion bonding. Accordingly, polyamide-based resins tend to have enhanced rigidity. Since the catheter tube according to the invention is formed from a polyamide-based resin having these characteristics, it is inferred that the catheter tube has a high burst strength.

Herein, the "polyamide-based resin" represents a polyamide resin, a polyamide elastomer, and/or a blend of these material.

A polyamide-based resin preferably used in the first polyamide layer is not particularly limited as long as the resin has an acid amide bond (-CO-NH-) in the main chain. The polyamide-based resin is typically produced by polymerization (homopolymerization) of a lactam or amino acid having a ring structure or by condensation polymerization of a dicarboxylic acid and a diamine in the presence of a preferable catalyst.

Examples of a homopolymerizable monomer include ε-caprolactam, undecane lactam, lauryl lactam, aminocaproic acid, 7-aminoheptanoic acid, 11-aminoundecanoic acid, 12-aminododecanoic acid, 9-aminononanoic acid, and piperidone.

In the condensation polymerization of a dicarboxylic acid and a diamine, examples of the dicarboxylic acid include adipic acid, sebacic acid, dodecane dicarboxylic acid, glutaric acid, terephthalic acid, 2-methylterephthalic acid, isophthalic acid, and naphthalenedicarboxylic acid. Examples of the diamine include tetramethylenediamine, hexamethylene diamine, nonamethylene diamine, decamethylene diamine, undecamethylene diamine, dodecamethylene diamine, p-phenylene diamine, and m-phenylenediamine.

As the polyamide-based resin, a polyamide resin (without a segment other than a polyamide) can be employed. Examples of the polyamide resin include NYLON (registered trademark) 4, 6, 7, 8, 11, 12, 6.6, 6.9, 6.10, 6.11, 6.12, 6T, 6/6.6, 6/12, 6/6T, and 6T/6I. Among these examples, NYLON 11 and NYLON 12 are particularly preferable as the polyamide resin.

As the polyamide-based resin, a polyamide elastomer is also applicable. Examples of the polyamide elastomer include a block copolymer of a polyamide (hard segment) and a polyether (soft segment), a block copolymer of a polyamide (hard segment) and a polyester (soft segment), a block copolymer of a polyamide (hard segment) and a polyether ester (soft segment), and more specifically, a block copolymer of NYLON (registered trademark) 11 and polytetramethylene glycol, and a block copolymer of NYLON 12 and polytetramethylene glycol.

Note that the terminal of the polyamide-based resin may be sealed by a carboxyl group, an amino group, or the like. One type of polyamide-based resin may be used independently, or two or more types of polyamide-based resins may be used in combination.

The polyamide-based resin may also employ a commercially available product such as DAIAMID (registered trademark) series (for example, L1640, L1840, L1940, L1940W, L2140, L2140W, and L2121), VESTAMID (registered trademark) series (which are all available from Daicel-Evonik Ltd.); RILSAMID (registered trademark) series, RILSAN (registered trademark) series, PEBAX (registered trademark) series (which are all available from Arkema); AMILAN (registered trademark) series (available from Toray Industries, Inc.); LEONA (registered trademark) series (available from Asahi Kasei Fibers Corp.); UBENYLON (registered trademark) series (available from UBE Corporation); RENY (registered trademark) series (available from Mitsubishi Engineering-Plastics Corporation); ZYTEL (registered trademark) series (available from DuPont); GRILAMID (registered trademark) series, and GRILFLEX (registered trademark) series (for example, EBG4530 NZ, ELG5930, ELG5660, ELG6260, and ELG4960) (which are all available from EMS-CHEMIE (Japan) Ltd.). Among these commercially available polyamide-based resins, one type of polyamide-based resin may be used independently, or two or more types of polyamide-based resins may be blended before use.

In a preferred embodiment of the invention, the polyamide-based resin included in the first polyamide layer is a polyamide resin.

The polyamide-based resin preferably has a weight average molecular weight of 10,000 to 500,000, and more preferably, 15,000 to 300,000. In the invention, the "weight average molecular weight" of the polyamide-based resin is a value measured by gel permeation chromatography (GPC) .

The first polyamide layer according to the invention contains a pressure resistance enhancer in a predetermined amount. The "pressure resistance enhancer" represents an agent to be added to enhance the burst strength (increase the pressure capacity) of a catheter tube.

In a preferred embodiment, the pressure resistance enhancer contains a base compound that contributes to enhancement of the burst strength and a dispersant for achieving desirable dispersibility in the polyamide-based resin.

Examples of the base compound include, but are not particularly limited to, inorganic pigment components like amorphous carbon such as carbon black; and metal compounds such as titanium oxide, barium sulfate, iron oxide (black iron oxide, yellow iron oxide, and red iron oxide), chromium oxide, ultramarine (ultramarine blue and ultramarine violet), nickel titanium yellow, Prussian
blue, Milori blue, cobalt blue, viridian, and molybdenum red. Furthermore, examples of the base compound of the pressure resistance enhancer also include organic pigment components like quinacridone-based pigments (such as quinacridone red), perylene-based pigments (such as perylene red), anthraquinone-based pigments (such as anthraquinone yellow), azo-based pigments (such as condensed azo-based yellow organic pigments), and phthalocyanine-based pigments (for example, halogenated phthalocyanines such as copper phthalocyanine and highly chlorinated copper phthalocyanine). Among these components, two or more types of base compounds are used in combination.

From a perspective of heat resistance during the extrusion or manufacturing of a catheter, the pressure resistance enhancer contains an inorganic pigment component as the base compound. Enhancement of the heat resistance makes it possible to heat a catheter at higher temperatures during annealing or fusion bonding in the manufacturing, which enhances the rigidity of a catheter tube and offers a higher burst strength to the catheter tube.

In addition to the enhancement of the burst strength, from a perspective of enhancing slidability of the surface of the first polyamide layer (inner layer), the pressure resistance enhancer contains amorphous carbon and a metal compound as the base compound. The "metal compound" herein refers to the metal compounds recited as the examples of the inorganic pigment components. According to the invention the pressure resistance enhancer contains carbon black and titanium oxide.

Catheter tubes containing carbon black, titanium oxide, ultramarine, perylene-based pigment, and phthalocyanine-based pigment as base compounds have an excellent burst strength. According to the invention the pressure resistance enhancer contains carbon black and titanium oxide. In another preferred embodiment, the pressure resistance enhancer contains carbon black, titanium oxide, and ultramarine. In another preferred embodiment, the pressure resistance enhancer contains carbon black, titanium oxide, ultramarine, and a perylene-based pigment. In another preferred embodiment, the pressure resistance enhancer contains a phthalocyanine-based pigment.

Carbon black, titanium oxide, and ultramarine are inorganic pigment components and have high heat resistance, enabling annealing and fusion bonding at high temperatures. Accordingly, a resulting catheter tube tends to have enhanced rigidity and enhances further in burst strength.

In addition to the base compound, the pressure resistance enhancer preferably contains a dispersant such as a surfactant. Adding a dispersant to the pressure resistance enhancer enhances dispersibility of the pressure resistance enhancer in the first polyamide layer, and it becomes easier for the pressure resistance enhancer to fulfill its function.

Examples of the dispersant contained in the pressure resistance enhancer include surfactants such as anionic surfactants (such as alkyl sulfates and fatty acid salts), cationic surfactants (such as aliphatic amine salts and quaternary ammonium salts), amphoteric surfactants, and nonionic surfactants (such as fatty acid esters and polyethylene glycol); and polymeric dispersants such polycarboxylic acid and amine salts thereof, polycarboxylic acid esters, polyurethanes, modified polyacrylates, polyester amides and amine salts thereof, alginic acid, polyvinyl alcohol, and carboxymethylcellulose.

Particularly, from not only a perspective of effectiveness in dispersing the base compound in the polyamide-based resin but also a perspective of enhanced safety, the pressure resistance enhancer preferably contains an anionic surfactant
as a dispersant, and more preferably, a metal salt of a higher fatty acid. Furthermore, from a similar perspective, the pressure resistance enhancer preferably contains at least one selected from the group consisting of, calcium laurate, calcium palmitate, calcium stearate, calcium oleate, zinc laurate, zinc palmitate, zinc stearate, and zinc oleate as a dispersant, and particularly preferably, the dispersant is calcium stearate and/or zinc stearate. Note that one type of dispersant may be used independently, or two or more types of dispersants may be used in combination.

In addition to the base compound and the dispersant, the pressure resistance enhancer may contain an additive such as a stabilizer.

The first polyamide layer according to the invention contains the pressure resistance enhancer in an amount more than 10 wt.% but equal to or less than 30 wt.% of total solids content of the first polyamide layer. In a case where the first polyamide layer contains the pressure resistance enhancer in an amount of 10 wt.% or less, the burst strength of a catheter tube is not sufficient and slidability of an inner layer surface is deteriorated. On the other hand, in a case where the first polyamide layer contains the pressure resistance enhancer in an amount more than 30 wt.%, the rigidity of a catheter tube becomes too high and cannot obtain sufficient flexibility, which may deteriorate the operability. Furthermore, in manufacturing a catheter tube, it is difficult to shape the first polyamide layer into a tube.

Moreover, in order to obtain a catheter (catheter tube) having not only an enhanced burst strength but also high flexibility and excellent operability while maintaining the desirable slidability of an inner layer surface, the first polyamide layer preferably contains the pressure resistance enhancer in an amount from 15 wt.% to 20 wt.% of total solids content of the first polyamide layer.

As described above, in a preferred embodiment of the invention, the pressure resistance enhancer contains a base compound and a dispersant. In the pressure resistance enhancer, a content ratio of the base compound to the dispersant is not particularly limited. For example, the pressure resistance enhancer is composed of 15 to 90 wt.% of the base compound (in a case where a plurality of base compounds is used, the total amount of the base compounds is within the range) and 85 to 10 wt.% of the dispersant (in a case where a plurality of dispersants is used, the total amount of the dispersants is within the range) (note that the total amount of the base compound and the dispersant is 100 wt.%). Using the pressure resistance enhancer having such compositions achieves desirable dispersibility of the pressure resistance enhancer in a layer and enhances the pressure resistance of a catheter tube. Preferably, the pressure resistance enhancer employed in the invention is composed of 20 to 70 wt.% of the base compound (in a case where a plurality of base compounds is used, the total amount of the base compounds is within the range) and 80 to 30 wt.% of the dispersant (in a case where a plurality of dispersants is used, the total amount of the dispersants is within the range) (note that the total amount of the base compound and the dispersant is 100 wt.%). More preferably, the pressure resistance enhancer is composed of 40 to 60 wt.% of the base compound (in a case where a plurality of base compounds is used, the total amount of the base compounds is within the range) and 60 to 40 wt.% of the dispersant (in a case where a plurality of dispersants is used, the total amount of the dispersants is within the range) (note that the total amount of the base compound and the dispersant is 100 wt.%).

In order to enhance the burst strength and slidability of the inner layer surface, the first polyamide layer preferably contains the base compound in an amount of 2.5 wt.% or more, more preferably 3 wt.% or more, still more preferably 5 wt.% or more, particularly preferably more than 5 wt.%, and most preferably 7 wt.% or more of total solids content. On the other hand, in order to achieve a catheter tube having sufficient flexibility and excellent operability, the first polyamide layer preferably contains the base compound in an amount of 25 wt.% or less, more preferably, 20 wt.% or less, particularly preferably, 15 wt.% or less, and most preferably, 12 wt.% or less of total solids content. Moreover, in order to enhance the burst strength, slidability of the inner layer surface, and flexibility in balance, an amount of the base compound relative to total solids content of the first polyamide layer is preferably within a range including any one of the above lower limits and any one of the above upper limits.

As described above, in the preferred embodiment of the invention, the pressure resistance enhancer contains amorphous carbon and a metal compound as base compounds. A content ratio between the amorphous carbon and metal compound is not particularly limited. For example, the base compound contained in the pressure resistance enhancer is composed of 0.1 to 60 wt.% of the amorphous carbon and 99.9 to 40 wt.% of the metal compound (in a case where a plurality of metal compounds is used, the total amount of the metal compounds is within the range) (note that the total amount of the amorphous carbon and the metal compound is 100 wt.%). Using the pressure resistance enhancer having such compositions, the catheter tube enhances in slidability of the inner layer surface and pressure resistance. Preferably, the base compound contained in the pressure resistance enhancer used in the invention is composed of 3 to 55 wt.% of the amorphous carbon and 97 to 45 wt.% of the metal compound (in a case where a plurality of metal compounds is used, the total amount of the metal compounds is within the range) (note that the total amount of the amorphous carbon and the metal compound is 100 wt.%).

In an embodiment where the pressure resistance enhancer contains amorphous carbon and a metal compound as base compounds, from a perspective of enhancing the burst strength and slidability of the inner layer surface, the first polyamide layer preferably contains amorphous carbon in an amount of 0.5 wt.% or more and 10 wt.% or less of total solids content of the first polyamide layer. From a similar perspective, the first polyamide layer preferably contains amorphous carbon in an amount more than 2.5 wt.% but equal to or less than 7.5 wt.% of total solids content of the first polyamide layer. Furthermore, from a similar perspective, the first polyamide layer preferably contains the amorphous carbon in an amount of 3 wt.% or more, and more preferably, 3.5 wt.% or more. On the other hand, in order to achieve a catheter tube having sufficient flexibility and excellent operability, the first polyamide layer preferably contains amorphous carbon in an amount of 7 wt.% or less, more preferably, 6 wt.% or less, and particularly preferably, 5 wt.% or less of total solids content. In addition, in order to enhance the burst strength, slidability of the inner layer surface, and flexibility in balance, the first polyamide layer preferably contains the amorphous carbon within a range including any one of the above lower limits and any one of the above upper limits with respect to total solids content of the first polyamide layer.

Note that whether the first polyamide layer contains amorphous carbon can be identified by laser Raman spectroscopy. In addition, an amount of the amorphous carbon contained can be measured by heating the polyamide-based resin for forming the first polyamide layer up to a temperature at which the resin is removed but not carbonized and by measuring the carbon residue content.

In an embodiment of the invention, the first polyamide layer preferably contains the dispersant in an amount of 3 wt.% or more, more preferably, more than 5 wt.%, and particularly preferably, 7 wt.% or more of total solids content of the first polyamide layer. On the other hand, in order to sufficiently achieve the effect of enhancing the burst strength, the first polyamide layer preferably contains the dispersant in an amount of 25 wt.% or less, more preferably, 20 wt.% or less, particularly preferably, 15 wt.% or less, and most preferably, 12 wt.% or less of total solids content.

In an embodiment of the invention, the first polyamide layer contains 70 wt.% or more and less than 90 wt.% (in solid content) of the polyamide-based resin, 3 to 25 wt.% (in solid content) of the base compound, and 3 to 25 wt.% (in solid content) of the dispersant in the total 100 wt.% of the first polyamide layer. In another embodiment, the first polyamide layer is composed of 70 wt.% or more and less than 90 wt.% (in solid content) of the polyamide-based resin, 3 to 25 wt.% (in solid content) of the base compound, and 3 to 25 wt.% (in solid content) of the dispersant (note that the total amount of the polyamide-based resin, base compound, and dispersant is 100 wt.%).

In a preferred embodiment of the invention, the first polyamide layer contains 70 wt.% or more and less than 90 wt.% (in solid content) of the polyamide-based resin, 3 to 20 wt.% (in solid content) of the base compound, and 3 to 20 wt.% (in solid content) of the dispersant in the total 100 wt.% of the first polyamide layer. In another preferred embodiment, the first polyamide layer is composed of 70 wt.% or more and less than 90 wt.% (in solid content) of the polyamide-based resin, 3 to 20 wt.% (in solid content) of the base compound, and 3 to 20 wt.% (in solid content) of the dispersant (note that the total amount of the polyamide-based resin, base compound, and dispersant is 100 wt.%).

In a more preferred embodiment of the invention, the first polyamide layer contains 80 wt.% to 85 wt.% (in solid content) of the polyamide-based resin, 5 to 10 wt.% (in solid content) of the base compound, and 5 to 10 wt.% (in solid content) of the dispersant in the total 100 wt.% of the first polyamide layer. In another more preferred embodiment, the first polyamide layer is composed of 80 wt.% to 85 wt.% (in solid content) of the polyamide-based resin, 5 to 10 wt.% (in solid content) of the base compound, and 5 to 10 wt.% (in solid content) of the dispersant (note that the total amount of the polyamide-based resin, base compound, and dispersant is 100 wt.%).

The pressure resistance enhancer is not particularly limited in shape and may be spherical or non-spherical. Examples of the non-spherical shape include various kinds of shapes, specifically, the shape of a polygonal prism such as triangular prism and rectangular prism, the shape of a cylinder, a cylindrical shape having a middle portion swelling further than both ends like a straw rice bag, the shape of a plate, what is called a cocoon shape having a constricted middle portion, what is called an associated spherical shape obtained by combining a plurality of particles, and the shape of a rugby ball. In order to enhance the slidability of the inner layer surface, the pressure resistance enhancer preferably has a spherical shape.

The pressure resistance enhancer is not particularly limited in structure and may have, for example, a hollow or solid structure. However, the pressure resistance enhancer preferably has a solid structure from a perspective of effectiveness in enhancing the burst strength. In addition, from a perspective of obtaining a high burst strength while maintaining desirable slidability of the inner layer surface, the pressure resistance enhancer preferably has a solid spherical shape.

The pressure resistance enhancer is not particularly limited in size, but the pressure resistance enhancer preferably includes primary particles having a mean particle diameter of, for example, more than 2.0 µm, more preferably, 3.0 µm or more, and particularly preferably, 5.0 µm or more. In addition, the pressure resistance enhancer preferably includes primary particles having a mean particle diameter of, for example, 20.0 µm or less, more preferably, 15.0 µm or less, and particularly preferably, 10.0 µm or less. Within these ranges, it is possible to contribute to enhancement of the burst strength while maintaining the desirable slidability of the inner layer surface. The mean particle diameter of the primary particles (mean primary-particle diameter) in the pressure resistance enhancer can be measured, for example, under conditions described in Examples.

The first polyamide layer preferably has a thickness (a radial thickness of an inner tube of the first polyamide layer) smaller than that of the second polyamide layer to be described (a radial thickness of an inner tube of the second polyamide layer). In an embodiment of the invention, there is provided a balloon catheter including a first polyamide layer having a thickness smaller than that of a second polyamide layer. The thickness of the first polyamide layer to the thickness of the second polyamide layer is in a ratio of, for example, 1 : 2 to 1 : 50 (thickness of the first polyamide layer : thickness of the second polyamide layer), and preferably, 1 : 3 to 1 : 8. The first polyamide layer preferably has a thickness of 5 to 30 µm, and more preferably, 7 to 20 µm. Setting the thickness of the first polyamide layer to 5 µm or more achieves desirable operability of a catheter (catheter tube) and setting the thickness to 30 µm or less enables a thin catheter tube with a small diameter.

To form the first polyamide layer containing the polyamide-based resin and the pressure resistance enhancer, for example, the polyamide-based resin and the pressure resistance enhancer may be kneaded in any ratio by a known technique to prepare pellets, and then, the pellets obtained may be used as a material of the first polyamide layer for coextrusion of a catheter tube.

### [Second Polyamide Layer]

In an embodiment of the invention, the second polyamide layer (outer layer) contains a polyamide-based resin. In a preferred embodiment of the invention, the second polyamide layer (outer layer) is composed of a polyamide-based resin. Since polyamide-based resins are flexible and have a high strength, they are employed as materials of an outer tube of a catheter or a balloon. Forming an outer layer of an inner tube, or a catheter tube, with a polyamide-based resin used in other members enhances compatibility between the members at the time of fusion bonding, which efficiently prevents the fracture of a catheter. In addition, employing a polyamide-based resin as a resin for forming an outer layer of an inner tube makes it possible to insert a catheter into a lumen without causing a kink even the lumen is greatly bent.

The polyamide-based resin used in the first polyamide layer and the molecular weight thereof are also applicable to the second polyamide layer. In order to obtain a catheter (catheter tube) having high flexibility and excellent operability in addition to desirable slidability and a desirable burst strength, the second polyamide layer preferably contains a polyamide resin and a polyamide elastomer. Particularly, in order to enhance the burst strength and the operability (flexibility) in balance, the first polyamide layer preferably contains a polyamide resin and the second polyamide layer preferably contains a polyamide resin and a polyamide elastomer (that is, the second polyamide layer contains a blend of the polyamide resin and the polyamide elastomer). In this embodiment, a mixing ratio (weight ratio) of the polyamide resin and the polyamide elastomer is preferably, but not particularly limited to, 50 : 50 to 95 : 5 (polyamide resin : polyamide elastomer, the same applies hereinafter), more preferably, 60 : 40 to 90 : 10, and still more preferably, 70 : 30 to 85 : 15. Furthermore, from a perspective of interlayer adhesiveness, the first polyamide layer and the second polyamide layer preferably employ the same polyamide-based resin.

The second polyamide layer preferably has a thickness larger than that of the first polyamide layer. In a case where a catheter tube is employed as an inner tube of a balloon catheter, a second polyamide layer with a larger thickness enhances the adhesiveness with respect to a distal tip and a balloon. More specifically, the second polyamide layer preferably has a thickness of 30 to 75 µm, and more preferably, 35 to 70 µm.

The catheter tube according to the invention can be formed by coextrusion using fine powder, coating dispersion, or pellets of the polyamide-based resin as a raw material (with regard to the first polyamide layer, the pressure resistance enhancer may also be used). Extrusion conditions may be optionally set by those skilled in the art. For example, a melting temperature may be set from 100 to 250°C. Furthermore, to enhance the burst strength, it is preferable to perform annealing after the extrusion. Annealing conditions are not particularly limited, but the annealing is preferably performed at a temperature of from 80 to 140°C for one to three hours.

### Examples

The effects of the invention will be described with reference to the following Examples and Comparative Examples. Note that the technical scope of the invention is not limited to the following Examples. Unless otherwise specified, each operation was performed at room temperature (25°C).

### [Example 1-1]

### (1) Preparation of Pellets of Composite Material

A polyamide resin (NYLON 12; DAIAMID L1940W; available from Daicel-Evonik Ltd.) and a pressure resistance enhancer 1 (containing 25 wt.% of carbon black, 25 wt.% of titanium oxide, 25 wt.% of calcium stearate, and 25 wt.% of zinc stearate (the total amount of the components was 100 wt.%); DAIREN Black PPB-0491 (available from DIC Corporation)) were compounded with a dual screw kneader to obtain a mixture of 80 wt.% of the polyamide resin and 20 wt.% of the pressure resistance enhancer 1 (the total amount of the polyamide resin and pressure resistance enhancer 1 was 100 wt.%). The polyamide resin and the pressure resistance enhancer 1 were kneaded twice to be mixed thoroughly. After the kneading, the resultant was extruded and cut, thereby obtaining pellets of a composite material 1 containing 20 wt.% of the pressure resistance enhancer 1.

The pressure resistance enhancer 1 had the form of a solid particle whose primary particles had a mean particle diameter of 6.2 µm. The mean particle diameter of the primary particles in the pressure resistance enhancer 1 was obtained by the following method.

A sample of the pressure resistance enhancer 1 in an amount of 0.005 g and 30 mL of 2-propanol as a dispersion medium were mixed, and the mean particle diameter was measured with a laser scattering particle size distribution analyzer (batch-type cell). The analyzer used was LA-960S (HORIBA Ltd.), the refractive index of the sample was 1.810, and the refractive index of the dispersion medium was 1.378.

### (2) Preparation of Tube

A copper wire was covered with a two-layer tube including an inner layer (a first polyamide layer composed of the composite material 1; thickness 17.5 µm) and an outer layer (a second polyamide layer; thickness 57.5 µm), followed by coextrusion. After the coextrusion, the copper wire was removed, and the tube was annealed at 130°C for two hours, thereby obtaining a tube 1-1 (inner diameter 0.43 mm × intermediate diameter 0.465 mm × outer diameter 0.58 mm). As a material of the second polyamide layer, a polyamide resin (NYLON 12; DAIAMID L1940W (available from Daicel-Evonik Ltd.)) was employed.

### [Comparative Example 1-1]

A comparative tube 1-1 was obtained in a manner similar to Example 3 described in JP 2016-174827 A (corresponding to US 2016/0271361 A).

### [Example 2-1]

### (1) Preparation of Pellets of Composite Material

Pellets of the composite material 1 were obtained in a manner similar to Example 1-1 (1).

### (2) Preparation of Tube

A tube 2-1 (inner diameter 0.43 mm × intermediate diameter 0.46 mm × outer diameter 0.58 mm) was obtained in a manner similar to Example 1-1 (2) except that a material of the outer layer (second polyamide layer) was changed to a polyamide-based resin containing a polyamide resin (NYLON 12; DAIAMID L1940W (available from Daicel-Evonik Ltd.)) and a polyamide elastomer (GRILFLEX ELG6260 (available from EMS-CHEMIE (Japan) Ltd.) in a 80 : 20 ratio.

### [Example 2-2]

### (1) Preparation of Pellets of Composite Material

Pellets of a composite material 2 were obtained in a manner similar to Example 1-1 (1) except that an amount of the polyamide resin contained was changed to 85 wt.% and an amount of the pressure resistance enhancer 1 contained was changed to 15 wt.%.

### (2) Preparation of Tube

A tube 2-2 was obtained in a manner similar to Example 2-1 (2) except that the pellets of the composite material 2 were used instead of the pellets of the composite material 1.

### [Comparative Example 2-1]

### (1) Preparation of Pellets of Composite Material

Pellets of a comparative composite material 1 were obtained in a manner similar to Example 1-1 (1) except that an amount of the polyamide resin contained was changed to 90 wt.% and an amount of the pressure resistance enhancer 1 contained was changed to 10 wt.%.

### (2) Preparation of Tube

A comparative tube 2-1 was obtained in a manner similar to Example 2-1 (2) except that the pellets of the comparative composite material 1 were used instead of the pellets of the composite material 1.

### [Example 3-1]

### (1) Preparation of Pellets of Composite Material

Pellets of a composite material 3 were obtained in a manner similar to Example 1-1 (1) except that the pressure resistance enhancer used in Example 1-1 (1) was changed to a different type of pressure resistance enhancer, a pressure resistance enhancer 2 (containing 33.4 wt.% of carbon black, 33.3 wt.% of calcium stearate, and 33.3 wt.% of zinc stearate (the total amount of the components was 100 wt.%); available from DIC Corporation).

The pressure resistance enhancer 2 had the form of a solid particle.

### (2) Preparation of Tube

A tube 3-1 was obtained in a manner similar to Example 2-1 (2) except that the pellets of the composite material 3 were used instead of the pellets of the composite material 1.

### [Example 3-2]

### (1) Preparation of Pellets of Composite Material

Pellets of a composite material 4 were obtained in a manner similar to Example 1-1 (1) except that the pressure resistance enhancer used in Example 1-1 (1) was changed to a different type of pressure resistance enhancer, a pressure resistance enhancer 3 (containing 33.4 wt.% of titanium oxide, 33.3 wt.% of calcium stearate, and 33.3 wt.% of zinc stearate (the total amount of the components was 100 wt.%); available from DIC Corporation).

The pressure resistance enhancer 3 had the form of a solid particle.

### (2) Preparation of Tube

A tube 3-2 was obtained in a manner similar to Example 2-1 (2) except that the pellets of the composite material 4 were used instead of the pellets of the composite material 1.

### [Example 3-3]

### (1) Preparation of Pellets of Composite Material

Pellets of a composite material 5 were obtained in a manner similar to Example 1-1 (1) except that the pressure resistance enhancer used in Example 1-1 (1) was changed to a different type of pressure resistance enhancer, a pressure resistance enhancer 4 (containing 13.1 wt.% of carbon black, 8.2 wt.% of titanium oxide, 41 wt.% of ultramarine, 4.9 wt.% of perylene red, 16.4 wt.% of calcium stearate, and 16.4 wt.% of zinc stearate (the total amount of the components was 100 wt.%); available from DIC Corporation).

The pressure resistance enhancer 4 had the form of a solid particle whose primary particles had a mean particle diameter of 2.8 µm.

### (2) Preparation of Tube

A tube 3-3 was obtained in a manner similar to Example 2-1 (2) except that the pellets of the composite material 5 were used instead of the pellets of the composite material 1.

### [Example 3-4]

### (1) Preparation of Pellets of Composite Material

Pellets of a composite material 6 were obtained in a manner similar to Example 1-1 (1) except that the pressure resistance enhancer used in Example 1-1 (1) was changed to a different type of pressure resistance enhancer, a pressure resistance enhancer 5 (containing 2.5 wt.% of carbon black, 35 wt.% of titanium oxide, 12.5 wt.% of ultramarine, 25 wt.% of calcium stearate, and 25 wt.% of zinc stearate (the total amount of the components was 100 wt.%); available from DIC Corporation).

The pressure resistance enhancer 5 had the form of a solid particle.

### (2) Preparation of Tube

A tube 3-4 was obtained in a manner similar to Example 2-1 (2) except that the pellets of the composite material 6 were used instead of the pellets of the composite material 1.

### [Example 3-5]

### (1) Preparation of Pellets of Composite Material

Pellets of a composite material 7 were obtained in a manner similar to Example 1-1 (1) except that the pressure resistance enhancer used in Example 1-1 (1) was changed to a different type of pressure resistance enhancer, a pressure resistance enhancer 6 (containing 0.3 wt.% of carbon black, 66.5 wt.% of titanium oxide, 16.6 wt.% of calcium stearate, and 16.6 wt.% of zinc stearate (the total amount of the components was 100 wt.%); available from DIC Corporation).

The pressure resistance enhancer 6 had the form of a solid particle.

### (2) Preparation of Tube

A tube 3-5 was obtained in a manner similar to Example 2-1 (2) except that the pellets of the composite material 7 were used instead of the pellets of the composite material 1.

### [Example 3-6]

### (1) Preparation of Pellets of Composite Material

Pellets of a composite material 8 were obtained in a manner similar to Example 1-1 (1) except that the pressure resistance enhancer used in Example 1-1 (1) was changed to a different type of pressure resistance enhancer, a pressure resistance enhancer 7 (containing 16.7 wt.% of copper phthalocyanine compound (Pigment Blue 15:3), 6.7 wt.% of copper phthalocyanine compound (Pigment Green 7), 25 wt.% of calcium stearate, and 51.6 wt.% of zinc stearate (the total amount of the components was 100 wt.%); available from DIC Corporation).

The pressure resistance enhancer 7 had the form of a solid particle.

### (2) Preparation of Tube

A tube 3-6 was obtained in a manner similar to Example 2-1 (2) except that the pellets of the composite material 8 were used instead of the pellets of the composite material 1.

### [Evaluation of Slidability]

The tubes obtained in Example 1-1 and Comparative Example 1-1 were set in a jig illustrated in Fig. 3. The tubes were filled with water. The length of each tube was set to 34 cm. A Ni-Ti alloy wire (φ 0.34 mm) was inserted through each tube, and a resistance was measured when retracting the wire. In order to ignore influence from the shape of a wire end, an average resistance obtained when the wire end passes through a straight section (length: 5 cm) was taken as the measured value. AUTOGRAPH (registered trademark) AG-X plus (Shimadzu Corporation) was used as a measuring device. The following Table 1 shows the results.

**[Table 1]**

| | Sliding resistance (gf) n = 5 |
|---|---|
| Example 1-1 (Inner layer: Polyamide resin 80 wt.%, Pressure resistance enhancer 20 wt.%) | 5.861 |
| Comparative example 1-1 (Inner layer: Modified polyolefin) | 5.901 |

Table 1 shows that the tube (catheter tube) according to the invention has desirable slidability comparable to a tube having an inner layer composed of a modified polyolefin. In other words, the invention enables a catheter tube having an inner tube (an inner surface) provided with desirable slidability. Note that the tubes (catheter tubes) obtained in Examples 2-1 and 2-2 also showed similar effects.

With regard to the tube obtained in Comparative Example 1-1, the copper wire was coated with a releasing agent in order to retract the copper wire after the coextrusion, but the tube obtained in Example 1-1 did not require such a process. Accordingly, even from a manufacturing viewpoint, the catheter tube according to the invention has excellent slidability.

### [Evaluation of Burst Strength]

### (1) Preparation of Evaluation Sample

Using the tubes obtained in Examples and Comparative Examples as inner tubes, evaluation samples simulating balloon catheters were prepared. Fig. 4 illustrates a rough image of an evaluation sample. In this evaluation sample, an inner tube 20 (each tube obtained in Examples and Comparative Examples) is inserted into an outer tube 30 (an outer tube shaft formed from a polyamide resin), and an end of an intermediate shaft 40 (formed from a polyamide resin) and an end (proximal side) of the outer tube 30 are subjected to thermal fusion bonding while overlapping one another. In the other end (distal side) of the outer tube 30, the inner tube 20 and the outer tube 30 are subjected to thermal fusion bonding to achieve airtightness. The intermediate shaft 40 has a pressurizing lumen (cavity), and the outside of the inner tube 20 is hydraulically pressurized from the intermediate shaft 40 through the outer tube 30. A sample with a broken balloon was used as an evaluation sample without a balloon since it cannot be evaluated in an appropriate manner.

### (2) Evaluation

In warm water at 37°C, a guidewire (ASAHI SION (registered trademark) Blue available from Asahi Intecc Co., Ltd.) was inserted through each inner tube. The guidewire was protruded from the distal side of each tube by 50 to 70 mm. In this state, each evaluation sample was pressurized at 1 atm (1 atm = 101325 Pa) for 30 seconds using the intermediate shaft, and then, depressurized. After that, the samples were pressurized with the pressure increasing step by step: for example, the samples were held at 2 atm for 30 seconds and depressurized, and then, held at 3 atm for 30 seconds and depressurized. In each depressurization, each catheter (evaluation sample) was moved while the guidewire was fixed, thereby measuring a pressure at which the guidewire could not pass through the inner tube in the proximal side. With regard to the evaluation of the burst strength, an appropriate evaluation (comparison) cannot be done under different minute conditions such as the degree of flatness or the like of the tube. For this reason, Examples and Comparative Examples are to be compared under the same conditions. Therefore, Example 1-1 was compared with Comparative Example 1-1 under the aforementioned conditions, and similarly, Examples 2-1 and 2-2 were compared with Comparative Example 2-2.

In comparing the results of Example 1-1 and Comparative Example 1-1, a ratio of the burst strength of the tube obtained in Example 1-1 to that of the tube obtained in Comparative Example was as 1.18 to 1.00. This result shows that the tube (catheter tube) according to the invention has an enhanced burst strength as compared with a tube having an inner layer composed of a modified polyolefin. In other words, the invention enables a catheter tube having an excellent burst strength. Note that the tube obtained in Comparative Example 1-1 also achieves a sufficient burst strength when used actually.

In addition, Examples 2-1 and 2-2 were compared with Comparative Example 2-1. The following Table 2 shows ratios of the burst strengths of the tubes obtained in Examples to that of the tube obtained in Comparative Example which is set to 1.00 and also shows values of the burst strengths obtained by the above evaluation method.

**[Table 2]**

| | Burst strength (atm) n = 3 | Ratio of burst strength |
|---|---|---|
| Example 2-1 (Pressure resistance enhancer: 20 wt.%) | 35.3 | 1.03 |
| Example 2-2 (Pressure resistance enhancer: 15 wt.%) | 35.3 | 1.03 |
| Comparative example 2-1 (Pressure resistance enhancer: 10 wt.%) | 34.3 | 1.00 |

As Table 2 shows, the burst strength tends to decrease when an amount of the pressure resistance enhancer is 10 wt.% of total solids content of the first polyamide layer. This result indicates that designing the first polyamide layer to contain the pressure resistance enhancer in an amount more than 10 wt.% enables a desirable burst strength.

### [Observation of Tube Surface]

The surface of the tube obtained in Example 1-1 was observed with a CCD camera (Fig. 5), and many fine structures (white parts in the photograph of Fig. 5) were found. Such fine structures indicate that there is no cohesion between the tube and the copper wire, and it is inferred that the fine structures contribute to desirable slidability.

This application is based on JP 2021-039903 A filed on March 12, 2021.

## Claims

1. A catheter comprising a catheter tube,
the catheter tube including a first polyamide layer and a second polyamide layer disposed on an outer surface of the first polyamide layer, and
the first polyamide layer containing a pressure resistance enhancer in an amount more than 10 wt.% but equal to or less than 30 wt.% of total solids content of the first polyamide layer;
wherein the pressure resistance enhancer contains amorphous carbon and a metal compound, wherein the amorphous carbon and metal compound are carbon black and titanium oxide.

2. The catheter according to claim 1, wherein the first polyamide layer contains the pressure resistance enhancer in an amount from 15 wt.% to 20 wt.% of total solids content of the first polyamide layer.

3. The catheter according to claim 1 or 2,
wherein the first polyamide layer contains the amorphous carbon in an amount more than 2.5 wt.% but equal to or less than 7.5 wt.% of total solids content of the first polyamide layer.

4. The catheter according to any one of claims 1 to 3, wherein the pressure resistance enhancer contains a primary particle having a mean particle diameter of 3.0 µm or more.

5. The catheter according to any one of claims 1 to 4, wherein the second polyamide layer contains a polyamide resin and a polyamide elastomer.

6. A balloon catheter including an outer tube having a lumen, an inner tube disposed in the lumen of the outer tube, and a balloon fixed to a distal side of the inner tube and a distal side of the outer tube,
the balloon containing a polyamide-based resin,
the inner tube including a first polyamide layer and a second polyamide layer disposed on an outer surface of the first polyamide layer, and
the first polyamide layer containing a pressure resistance enhancer in an amount more than 10 wt.% but equal to or less than 30 wt.% of total solids content of the first polyamide layer;
wherein the pressure resistance enhancer contains amorphous carbon and a metal compound, wherein the amorphous carbon and metal compound are carbon black and titanium oxide.

7. The balloon catheter according to claim 6, wherein the first polyamide layer contains the pressure resistance enhancer in an amount from 15 wt.% to 20 wt.% of total solids content of the first polyamide layer.

8. The balloon catheter according to claim 6 or 7,
wherein
the first polyamide layer contains the amorphous carbon in an amount more than 2.5 wt.% but equal to or less than 7.5 wt.% of total solids content of the first polyamide layer.

9. The balloon catheter according to any one of claims 6 to 8, wherein the pressure resistance enhancer contains a primary particle having a mean particle diameter of 3.0 µm or more.

10. The balloon catheter according to any one of claims 6 to 9, wherein the second polyamide layer contains a polyamide resin and a polyamide elastomer.

## Patentansprüche

1. Katheter, umfassend einen Katheterschlauch, wobei der Katheterschlauch eine erste Polyamidschicht und eine zweite Polyamidschicht umfasst, die auf einer äußeren Oberfläche der ersten Polyamidschicht angeordnet ist, und
wobei die erste Polyamidschicht einen Druckbeständigkeits-Verstärker in einer Menge von mehr als 10 Gew.-%, jedoch gleich oder weniger als 30 Gew.-% des Gesamtfeststoffgehalts der ersten Polyamidschicht enthält;
wobei der Druckbeständigkeits-Verstärker amorphen Kohlenstoff und eine Metallverbindung enthält, wobei der amorphe Kohlenstoff und die Metallverbindung Ruß und Titanoxid sind.

2. Katheter nach Anspruch 1, wobei die erste Polyamidschicht den Druckbeständigkeits-Verstärker in einer Menge von 15 Gew.-% bis 20 Gew.-% des Gesamtfeststoffgehalts der ersten Polyamidschicht enthält.

3. Katheter nach Anspruch 1 oder 2, wobei die erste Polyamidschicht den amorphen Kohlenstoff in einer Menge von mehr als 2,5 Gew.-%, jedoch gleich oder weniger als 7,5 Gew.-% des Gesamtfeststoffgehalts der ersten Polyamidschicht enthält.

4. Katheter nach einem der Ansprüche 1 bis 3, wobei der Druckbeständigkeits-Verstärker ein Primärpartikel enthält, das einen mittleren Partikeldurchmesser von 3,0 µm oder mehr aufweist.

5. Katheter nach einem der Ansprüche 1 bis 4, wobei die zweite Polyamidschicht ein Polyamidharz und ein Polyamidelastomer enthält.

6. Ballonkatheter, der einen Außenschlauch umfasst, der ein Lumen, einen in dem Lumen des Außenschlauchs angeordneten Innenschlauch und einen an einer distalen Seite des Innenschlauchs und einer distalen Seite des Außenschlauchs befestigten Ballon aufweist,
wobei der Ballon ein Harz auf Polyamidbasis enthält,
wobei der Innenschlauch eine erste Polyamidschicht und eine zweite Polyamidschicht umfasst, die auf einer äußeren Oberfläche der ersten Polyamidschicht angeordnet ist, und
wobei die erste Polyamidschicht einen Druckbeständigkeits-Verstärker in einer Menge von mehr als 10 Gew.-%, jedoch gleich oder weniger als 30 Gew.-% des Gesamtfeststoffgehalts der ersten Polyamidschicht enthält;
wobei der Druckbeständigkeits-Verstärker amorphen Kohlenstoff und eine Metallverbindung enthält, wobei der amorphe Kohlenstoff und die Metallverbindung Ruß und Titanoxid sind.

7. Ballonkatheter nach Anspruch 6, wobei die erste Polyamidschicht den Druckbeständigkeits-Verstärker in einer Menge von 15 Gew.-% bis 20 Gew.-% des Gesamtfeststoffgehalts der ersten Polyamidschicht enthält.

8. Ballonkatheter nach Anspruch 6 oder 7,
wobei
die erste Polyamidschicht den amorphen Kohlenstoff in einer Menge von mehr als 2,5 Gew.-%, jedoch gleich oder weniger als 7,5 Gew.-% des Gesamtfeststoffgehalts der ersten Polyamidschicht enthält.

9. Ballonkatheter nach einem der Ansprüche 6 bis 8, wobei der Druckbeständigkeits-Verstärker ein Primärpartikel enthält, das einen mittleren Partikeldurchmesser von 3,0 µm oder mehr aufweist.

10. Ballonkatheter nach einem der Ansprüche 6 bis 9, wobei die zweite Polyamidschicht ein Polyamidharz und ein Polyamidelastomer enthält.

## Revendications

1. Cathéter comprenant un tube de cathéter, le tube de cathéter comprenant une première couche de polyamide et une seconde couche de polyamide disposée sur une surface extérieure de la première couche de polyamide, et
la première couche de polyamide contenant un agent améliorant la résistance à la pression en une quantité supérieure à 10 % en poids mais inférieure ou égale à 30 % en poids de la teneur totale en solides de la première couche de polyamide ;
l'agent améliorant la résistance à la pression contenant du carbone amorphe et un composé métallique, le carbone amorphe et le composé métallique étant du noir de carbone et de l'oxyde de titane.

2. Cathéter selon la revendication 1, dans lequel la première couche de polyamide contient l'agent améliorant la résistance à la pression en une quantité de 15 % en poids à 20 % en poids de la teneur totale en solides de la première couche de polyamide.

3. Cathéter selon la revendication 1 ou 2, dans lequel la première couche de polyamide contient le carbone amorphe en une quantité supérieure à 2,5 % en poids mais inférieure ou égale à 7,5 % en poids de la teneur totale en solides de la première couche de polyamide.

4. Cathéter selon l'une quelconque des revendications 1 à 3, dans lequel l'agent améliorant la résistance à la pression contient une particule primaire ayant un diamètre moyen de particule de 3,0 µm ou plus.

5. Cathéter selon l'une quelconque des revendications 1 à 4, dans lequel la seconde couche de polyamide contient une résine polyamide et un élastomère polyamide.

6. Cathéter à ballonnet comprenant un tube extérieur comportant un lumen, un tube intérieur disposé dans le lumen du tube extérieur et un ballonnet fixé à un côté distal du tube intérieur et à un côté distal du tube extérieur,
le ballon contenant une résine à base de polyamide,
le tube intérieur comprenant une première couche de polyamide et une seconde couche de polyamide disposée sur une surface extérieure de la première couche de polyamide, et
la première couche de polyamide contenant un agent améliorant la résistance à la pression en une quantité supérieure à 10 % en poids mais inférieure ou égale à 30 % en poids de la teneur totale en solides de la première couche de polyamide ;
l'agent améliorant la résistance à la pression contenant du carbone amorphe et un composé métallique, le carbone amorphe et le composé métallique étant du noir de carbone et de l'oxyde de titane.

7. Cathéter à ballonnet selon la revendication 6, dans lequel la première couche de polyamide contient l'agent améliorant la résistance à la pression en une quantité de 15 % en poids à 20 % en poids du total des solides de la première couche de polyamide.

8. Cathéter à ballonnet selon la revendication 6 ou 7,
dans lequel
la première couche de polyamide contient le carbone amorphe en une quantité supérieure à 2,5 % en poids mais inférieure ou égale à 7,5 % en poids de la teneur totale en solides de la première couche de polyamide.

9. Cathéter à ballonnet selon l'une quelconque des revendications 6 à 8, dans lequel l'agent améliorant la résistance à la pression contient une particule primaire ayant un diamètre moyen de particule de 3,0 µm ou plus.

10. Cathéter à ballonnet selon l'une quelconque des revendications 6 à 9, dans lequel la seconde couche de polyamide contient une résine polyamide et un élastomère polyamide.
